# EUROPEAN PATENT APPLICATION

(11) **EP 4 752 900 A1**
(43) Date of publication of application: **03.06.2026**
(21) Application number: 24216866.4
(22) Date of filing: 02.12.2024
(51) Int. Cl.: G16H 30/20

(54) **METHOD OF CONFIGURING A CT IMAGE DATASET**

(71) Applicant: Koninklijke Philips N.V., 5656 AE Eindhoven (NL)
(72) Inventor: WIEMKER, Rafael, Eindhoven (NL); SCHMITT, Holger, Eindhoven (NL); SCHULZ, Heinrich, Eindhoven (NL); NETSCH, Thomas, Eindhoven (NL)
(74) Representative: Philips Intellectual Property & Standards

(57) **Abstract**

A method for configuring a CT image data set to improve interoperability without increasing data package size. The method comprises compiling CT image data into a DICOM data object with DICOM metadata, and wherein the compiled CT image data includes both images compressed in a form that is DICOM readable and images compressed in a form that is not DICOM readable.

## Description

### FIELD OF THE INVENTION

This invention relates to the field of data handling in CT imaging systems, for example the storage, transmission and viewing of CT image data.

### BACKGROUND OF THE INVENTION

Spectral CT imaging, also known as multi-energy CT, is an advanced form of computed tomography that enables distinguishing between different materials within the scanned object. Unlike conventional CT, which measures the overall attenuation of x-rays passing through a scanned body using a single energy spectrum, spectral CT concurrently collects x-ray measurement data across multiple energy channels. This is achieved by utilizing detectors sensitive to different energy levels or by rapidly switching the x-ray tube voltage between a series of distinct energy settings. The x-ray measurement data is therefore not only a function of spatial coordinates but also of the energy spectrum of the x-rays, creating multi-channel projection dataset known as spectral CT projection data.

In the spectral CT process, this spectral CT projection data is first acquired, capturing information as to the attenuation of x-rays of varying energies by the scanned object across different projection paths. This data forms the basis for advanced image reconstruction techniques that take advantage of the different energy dependencies of the x-ray attenuation properties of different materials. By applying material decomposition algorithms to the spectral CT projection data, it is possible to separate and quantify specific materials within the scanned object. After material decomposition is applied, image reconstruction algorithms may be applied which results in the creation of material images, which may represent different tissues, contrast agents, or other substances within the object. Material images can also include so-called photo images or scatter images, which represent the relative contribution to x-ray attenuation as a function of spatial position of each of Photoelectric absorption and Compton Scatter. Material maps represent the spatial distribution of particular materials through the scanned body.

The large variety of possible material images that can be generated using spectral CT imaging results in a potentially large amount of data that needs to be stored or transmitted from the CT imaging system to an image viewer or image storage system (e.g. PACS system). This creates problems in terms of storage capacity requirements and data transfer capacity, including time taken for data transfer.

These problems can be mitigated partly by applying data compression techniques to the image data.

Data compression applied to an image can result in reduction of the matrix size of the image. Matrix size is the number of pixels in the reconstructed CT image grid. It is typically expressed as a two-dimensional array, represented by the number of rows and columns of pixels (e.g., 512 x 512, 1024 x 1024). For example, a matrix size of 512 x 512 means there are 512 pixels along the width (columns) and 512 pixels along the height (rows) of the image. A voxel (volumetric pixel) represents a volume element in the 3D reconstruction, which corresponds to the pixel area in 2D and the slice thickness. The voxel size is influenced by the matrix size, the field of view (FOV), and the slice thickness:
Matrix size directly influences the spatial resolution of the CT image. A larger matrix size (e.g., 1024 x 1024) provides higher spatial resolution. A smaller matrix size (e.g., 256 x 256) results in lower spatial resolution.

Commonly used CT scanners yield images with matrix sizes of 512 x 512 for routine diagnostic imaging. For specific applications requiring high spatial resolution (e.g., imaging of small bones, ear structures, or small vascular details), matrix sizes of 1024 x 1024 or higher may be used.

A variety of different image compression protocols and procedures are known in the field which can be used to reduce the matrix size of reconstructed CT images for storage or transmission. Two classifications are lossless and lossy compression.

Lossless compression reduces the file size without losing any information from the original image. When the image is decompressed, it is identical to the original, with no loss in quality or detail. Examples techniques include Run-Length encoding (RLE), Huffinan Coding, Lempel-Ziv-Welch (LZW). Lossless compression is commonly used in medical imaging (e.g. DICOM) because it avoids loss of image integrity.

Lossy compression reduces file size by removing some of the image data, which results in a loss of quality. The decompressed image is not identical to the original but is often visually similar. Lossy compression can achieve greater reduction in data size (matrix size) than lossless compression. Examples techniques include transform coding, quantization and wavelet transforms. Lossy compression is commonly used for web images, digital photography, and other uses where a slight loss in image integrity is acceptable to achieve reduced file size.

DICOM (Digital Imaging and Communications in Medicine) is a globally recognized standard used for handling, storing, printing, and transmitting information in medical imaging. It includes a file format definition and a network communications protocol, which enables the integration of medical imaging devices such as scanners, servers, workstations, printers, network hardware, and Picture Archiving and Communication Systems (PACS) from multiple manufacturers.

The DICOM standard defines a data structure for the storage and communication of medical images and related information. The DICOM data structure allows for a uniform way to store and access image data. The DICOM data structure includes a DICOM file which acts as a container for the data stored by the DICOM data structure and includes a header and image data. The header, or preamble, contains DICOM metadata, including patient information, study details, and equipment used. The image data follows the header, containing the pixel data of the image itself. The metadata is stored as data elements with specific tags that make it easy to search, retrieve, and organize image data within a PACS or other medical imaging systems.

DICOM can read and store images compressed in a limited set of compression formats, which include: JPEG (Lossy), JPEG-LS (Lossless), JPEG 2000 (Lossy and Lossless), Run-Length Encoding (RLE).

PACS (Picture Archiving and Communication System) is a medical imaging technology used for storing, retrieving, managing, and sharing medical images electronically. PACS eliminates the need for manually handling film-based images and provides remote access to images, streamlining the workflow of medical professionals.

PACS uses the DICOM standard to store images. Each image is saved as a DICOM container file, which includes the image data and associated metadata. The DICOM standardized format ensures that images from different modalities and manufacturers can be stored in a unified format. DICOM also provides the protocols for transferring images from imaging devices to PACS and between PACS and workstations. The DICOM communication protocol is based on TCP/IP, enabling devices to communicate over local or wide-area networks. DICOM supports query/retrieve operations, allowing users to search for and retrieve specific images or series from the PACS based on various criteria (e.g., patient name, study date). DICOM ensures that imaging equipment and PACS from different manufacturers can work together, as all adhere to the same standard. This interoperability is crucial for integrating imaging systems across different departments and institutions.

Therefore the DICOM protocol limits the extent to which data which is to be DICOM-readable can be compressed. This poses problems in the context of spectral CT because the limited range of DICOM-compatible compression formats do not allow data size to be reduced sufficiently for the large amounts of possible material image data to be efficiently stored or transferred.

A solution is to compress image data using a compression format which reduces data size to a greater extent and then to store that image data in a proprietary data structure which can be read by a proprietary image viewer console. However, this causes a lack of interoperability with standard DICOM systems since the proprietary format cannot be read by these.

There is required an improved way of configuring a CT image dataset for transfer or storage in a way that allows for more spectral image data to be included in the image dataset without significantly increasing data package size and which still allows for interoperability with DICOM systems.

### SUMMARY OF THE INVENTION

The invention is defined by the claims.

According to examples in accordance with an aspect of the invention, there is provided a CT image dataset configured in a DICOM data structure. The CT image dataset comprises post-reconstruction CT image data generated from spectral CT projection data. The spectral CT projection data comprises x-ray measurement data for a plurality of energy channels. The post-reconstruction CT image data includes one or more material images formed from reconstruction of the spectral CT projection data after material decomposition has been applied.

The DICOM data structure comprises: a container; DICOM metadata; and a set of data packages, the set of data packages including a first data package and a second data package. The first data package comprises (A) a set of one or more first images. Each first image of the set of one or more first images is a post-reconstruction CT image which is compressed in a first compression format. The first compression format is not DICOM readable. The second data package comprises (B) a second image. The second image is a post-reconstruction CT image which is compressed in a second compression format, wherein the second compression format is DICOM readable.

The first and second compression formats may be such that the second image is compressed to a lesser level of compression than each of the one or more first images. A lesser level of compression means that the second compression format results in less reduction in the data size of the image compared to the first compression format. In some embodiments, the first compression format is a lossy compression format. In other words, the one or more material images are compressed with a lossy compression method, so are lower resolution (and therefore cannot be read by DICOM).

The invention is based on the concept of packaging CT image data into a DICOM data structure (i.e. comprising DICOM metadata, making it able to be handled by DICOM systems), wherein the CT image data packaged in the DICOM data structure includes some image objects which are compressed a way that is DICOM readable and some image objects which are compressed a way that is not DICOM readable. The DICOM readable image can be displayed on a DICOM console and/or stored in a PACS, while the non-DICOM images can be used by a proprietary image reader for more advanced functions, e.g. generating custom spectral or material images.

By way of example the DICOM-readable second image may be a MonoE or pseudo-conventional image because these are the most widely recognized and understood image types by clinicians. By way of example, the one or more first images may include a photo image or scatter image. These are less widely recognized or understood but (for reasons that will be explained later) these allow, in combination with the MonoE or pseudo-conventional image in the second data package, for reconstruction of a range of different possible material maps. Thus, this is an efficient data transfer method in the context of spectral CT since it minimizes data structure size but includes sufficient spectral information to reconstruct a range of different spectral maps on the image viewer side. One or more precomputed material maps may additionally be included in the first data package and compressed in the first compression format. After data transfer or storage these can be directly displayed without an image reconstruction operation, thus saving time.

A DICOM-readable compression format means that the second image is compatible with the DICOM standard. In other words, the second image (e.g. conventional or MonoE image) is at least suitable to be read by an image handling software which operates according to the DICOM standard, e.g. DICOM image reading software and/or PACS image storage software. A compression format which is not DICOM-readable means that each of the one or more first images is non-compatible with the DICOM standard. In other words, the one or more material images are compressed in a non-DICOM-readable format

In some embodiments, the second image is an image formed from a reconstruction applied to a weighted combination of two or more spectral channels of the spectral CT projection data, wherein the two or more spectral channels are two or more energy channels of the measurement data or two or more spectral channels of the CT projection data after material decomposition has been applied. For example, and as noted above, the second image may be MonoE image, or may be a pseudo-conventional image. The pseudo-conventional image may be an image formed from a reconstruction applied to a weighted combination of two or more of the measurement data energy channels.

In some embodiments, at least one of the set of one or more first images is one of: a Compton Scatter image or a Photoelectric effect image.

In a preferred set of embodiments, at least one of the set of one or more first images is one of: a Compton Scatter image or a Photoelectric effect image; and the second image is an image formed from a reconstruction applied to a weighted combination of two or more spectral channels of the spectral CT projection data, wherein the two or more spectral channels are two or more energy channels of the measurement data or two or more spectral channels of the CT projection data after material decomposition has been applied. This combination of objects allows for generation of any desired spectral channel and/or material map using the pseudo-conventional or MonoE image, in combination with the photo or scatter image.

Additionally or alternatively, at least one of the one or more first images comprises a material map, and preferably wherein the one or more first images include a plurality of different material maps.

In some embodiments, the first compression format comprises one or more decomposition levels of a Laplacian pyramid decomposition applied to a starting CT image. In some embodiments, the first compression format comprises a lowest resolution decomposition level of the Laplacian pyramid decomposition applied to the starting CT image. Laplacian-pyramid based compression is a lossy compression method and is not DICOM-readable.

In some embodiments, the data structure further includes all decomposition levels of a Laplacian pyramid decomposition of the second image. For reasons that will be explained later, during reconstruction of the compressed first images, the decomposition layers of the second image can be used to reconstruct a better resolution version of each of the original first images, i.e. it can be used to reduce some of the data loss incurred in the original downsampling process.

Another aspect of the invention is a method of forming or of configuring a CT image dataset. The CT image dataset comprises post-reconstruction CT image data generated from spectral CT projection data. The spectral CT projection data comprises x-ray measurement data for a plurality of energy channels. The post-reconstruction CT image data includes one or more material images formed from reconstruction of the CT projection data after material decomposition has been applied,

The method comprises a) obtaining one or more first images, wherein each of the one or more first images is a post-reconstruction CT image. The method further comprises b) obtaining a second image; wherein the second image is a post-reconstruction CT image. The method further comprises c) compressing the one or more first images in a first compression format, wherein the first compression format is not DICOM-readable. The method further comprises d) compressing the second image in a second compression format, wherein the second compression format is DICOM-readable. The method further comprises e) packaging the one or more first images to form a first data package. The method further comprises f) packaging the second image to form a second data package. The method further comprises g) compiling a DICOM data structure, wherein the DICOM data structure includes a container, DICOM metadata, the first data package and the second data package.

In some embodiments, the steps (a)-(g) are performed at a first processing device; and wherein the method further comprises transferring the DICOM data structure to a second processing device. This thus provides a method of communicating image data.

In some embodiments, the method further comprises, at the second processing device: receiving the DICOM data structure; and performing one or more of the following:
- extracting the one or more first images, decompressing at least one of the one or more first images, and displaying at least one of the one or more first images using an image viewer which is operable to read and display images which are in a compression format which is not DICOM readable;
- extracting the second image, decompressing the second image, and displaying the second image using a DICOM image viewer; and
- storing the received DICOM data package in a DICOM-compatible PACS system.

In some embodiments, the method may further comprise extracting the second image from the data structure; and decomposing the second image into two or more projection domain spectral channel components, wherein the two or more projection domain spectral channels are two or more energy channels of the measurement data or two or more spectral channels of the CT projection data after material decomposition has been applied, e.g. photo and scatter channels.

In some embodiments, the second image is an image formed from a reconstruction applied to a weighted combination of two or more spectral channels of the spectral CT projection data, wherein the two or more spectral channels are two or more energy channels of the measurement data or two or more spectral channels of the CT projection data after material decomposition has been applied, and wherein the set of one or more first images includes a photoelectric effect image or a Compton Scatter image. Here, the method may further comprise a supplementary reconstruction operation comprising processing the second image and the photoelectric effect or scatter image to yield a material map image.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of the processing flow in an example CT imaging system;
Fig. 2 is a block diagram of an example CT image data set configured as a DICOM data structure in accordance with one or more embodiments;
Fig. 3 is a block diagram of a preferred embodiment of a CT image data set configured as a DICOM data structure;
Fig. 4 illustrates compression of CT images in accordance with a first and second compression technique;
Fig. 5 illustrates packaging compressed first and second images into first and second data packages;
Fig. 6 illustrates compiling a DICOM data structure with first and second data packages;
Fig. 7 illustrates an example data transfer method;
Fig. 8 illustrates a range of options for handling a data structure transferred in a data transfer method depending upon if a DICOM image reader or a non-DICOM image reader is available;
Fig. 9 illustrates a process for reconstructing any arbitrary material map using a combination of a first image being a photo or scatter image and a second image being an image formed from reconstruction applied to a weighted combination of projection domain spectral channels, e.g. a MonoE image or a pseudo-conventional image; and
Fig. 10 shows an example image compression technique utilizing Laplacian pyramid decomposition.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for configuring a CT image data set to improve interoperability without increasing data package size. The method comprises compiling CT image data into a DICOM data object with DICOM metadata, and wherein the compiled CT image data includes both images compressed in a form that is DICOM readable and images compressed in a form that is not DICOM readable.

Embodiments of the invention are applicable within the context of a CT imaging environment which comprises a CT imaging system for acquiring CT image data and for processing acquired raw CT image data to reconstruct one or more images.

Embodiments of the invention have particular applicability within the context of a spectral CT imaging environment.

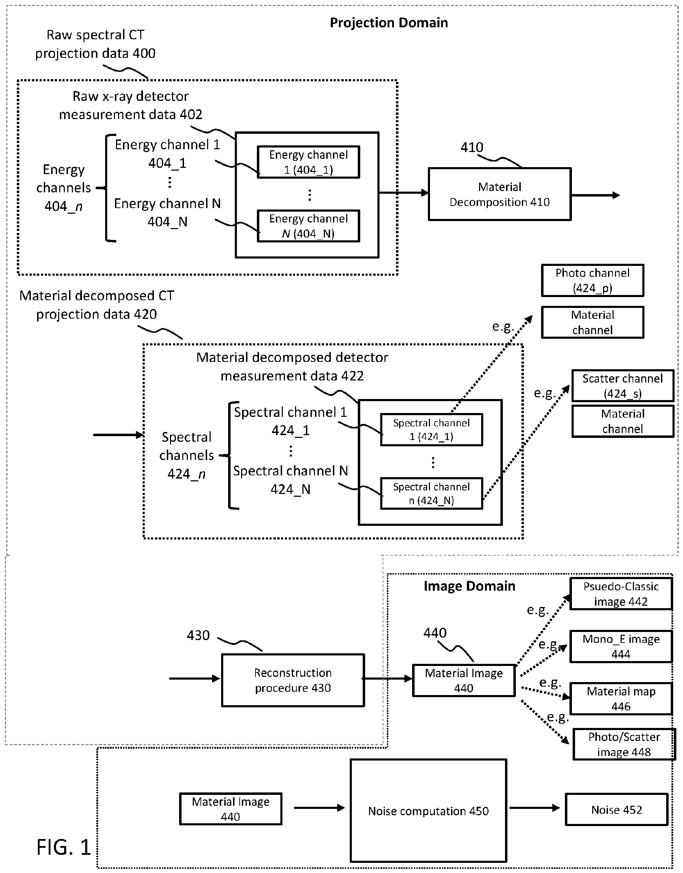

For purposes of context, Fig. 1 outlines schematically the data processing flow of an example spectral CT imaging system. These details will be well known to the skilled person in this field and are outlined to provide context to the embodiments of the invention discussed later.

A CT imaging system comprises an x-ray source and an x-ray detector, these being mounted to a rotatable gantry, diametrically opposite to one another.

The x-ray detector outputs raw x-ray measurement data 402. The x-ray detector is a multi-energy x-ray detector. The multi-energy x-ray detector detects x-ray radiation in two different energy ranges, each of these forming one of a set of two or more energy channels 404_1,... ,404_N of the acquired raw x-ray detector measurement data 402. The two or more energy channels may be referred to generically by the reference numeral 404_n.

The x-ray detector may be a dual-energy detector and consequently the raw x-ray detector measurement data 402 may consist of two energy channels 404n. These may be referred to as high and low energy channels.

The x-ray detector may be a photon counting x-ray detector. Photon counting x-ray detectors can differentially detect photons of different energies and bin them into a set of energy bins. Typically photon counting x-ray detectors allow for acquiring raw detector measurement data in a greater number of different energy channels than more traditional systems that, for example, are only dual energy and thus have only two energy channels.

Additionally or alternatively the x-ray source may be a multi-energy x-ray source. The multi-energy x-ray source is operable to emit an x-ray radiation output with an x-ray energy which changes as a function of time. For example, it may progress cyclically through a sequence of two or more x-ray emission energy levels, each spanning a respective duty cycle period. The x-ray detector detects x-ray radiation in temporal channels, each temporal channel spanning one of the duty cycle periods. Each temporal channel therefore corresponds to one of the set of two or more x-ray source energy levels, thereby resulting in raw x-ray detector measurement data 402 which comprises two or more energy channels 404_n.

If the system is a dual-energy system, the multi-energy x-ray source may be a kVp-switching x-ray source.

The raw x-ray detector measurement data 402 is processed with a material decomposition operation 410. Material decomposition in spectral CT comprises converting the energy channels of the detector data into spectral channels, where the spectral channels correspond to the contribution made to x-ray attenuation by particular materials (e.g. iodine, water, calcium) or particular attenuation phenomena (Compton scatter, Photoelectric absorption). The material decomposition might instead be referred to as spectral decomposition. Material decomposition is based on the fact that different materials attenuate x-rays differently depending upon their energy. The concept is to express the raw x-ray detector energy channel data as a linear combination of the attenuation coefficients of basis materials. Different pre-existing algorithms exist depending upon the particular set of basis materials that are chosen. The relevant algorithm often can be stored as a simple lookup table that allows for linear mapping between a set of raw detector energy channel values and corresponding values for a set of chosen basis materials or attenuation phenomena.

The material decomposition operation 410 comprises applying one or more material or spectral decomposition algorithms to the raw x-ray detector measurement data 402. The output of the material decomposition operation 410 is a set of material decomposed CT projection data 420. This include a material decomposed version 422 of the detector measurement data which comprises two or more spectral channels 424_1,...424_N. The two or more spectral channels may be referred to generically in this disclosure by the reference numeral 424_n. The two or more spectral channels correspond to the chosen two or more basis materials or basis attenuation phenomena. In one example, the two or more spectral channels may comprise: a Photoelectric effect channel 424_p (which represents the effective contribution to attenuation along the relevant projection line of the photoelectric absorption effect), and a Compton Scatter channel 424_s (which represents the effective contribution to attenuation along the relevant projection line of Compton scatter). These are the two fundamental x-ray attenuation phenomena within any material that might be imaged. These channels are highly useful because they allow to reconstruct a range of other spectral channels, including a variety of different material channels. The two or more spectral channels may additionally or alternatively include one or more material channels. Possible material channels which can be obtained by material decomposition include for instance water channel, iodine channel, bone channel, fat channel, soft tissue channel, calcium channel.

After material decomposition, an image reconstruction procedure 430 is applied. The image reconstruction procedure 430 comprises applying one or more image reconstruction algorithms to the materially decomposed detector measurement data 422.

The output of the image reconstruction procedure 430 is one or more material images 440. A material image in the context of this disclosure simply means an image which maps the distribution of a material (e.g. calcium, iodine) or of an attenuation phenomenon (e.g. photoelectric effect, Compton scatter) across a slice or volume of the imaged body. It is referred to as a material image because it is an image generated from reconstruction applied to the measurement data after a material decomposition procedure is applied. However, it might alternatively be referred to as a spectral image for example.

Different reconstruction algorithms can be applied as part of the image reconstruction procedure 430 to yield different types of material image 440.

One type of material image 440 which may be reconstructed is a so-called pseudo-conventional image 442 (sometime also referred to as pseudo-classical image). A pseudo-conventional image 442 is generated by combining the spectral channel data 422 in a way that mimics the appearance of a conventional single-energy CT scan. This involves processing the spectral channels 424_n of the materially decomposed detector measurement data 422 (e.g. a Compton and Photo channel) and/or processing the energy channels 404_n of the raw x-ray detector measurement data 402 to create an image that appears similar to those obtained from standard CT, using weighted averages or other algorithms that blend the data from multiple energies. The main purpose of a pseudo-conventional image is to provide a familiar image representation to clinicians who are accustomed to conventional CT. Pseudo-conventional images are often used as a reference or baseline image in a spectral CT exam, alongside other advanced images (such as MonoE, iodine maps, etc.), to provide a comprehensive diagnostic view. There are known algorithms for computing pseudo-conventional images. These images may be formed by applying image reconstruction to a weighted combination of the raw x-ray detector measurement data energy channels 404_n, and/or to a weighted combination of the material decomposed detector measurement data spectral channels 424_n.

Another type of material image 440 which may be reconstructed is a monoenergetic (MonoE) image 444. A monoenergetic image represents what an image (of a slice or volume of the image body) would look like if it were acquired using x-rays of a single energy level (or very narrow energy range). In spectral CT, the data acquired at multiple energy levels are used to reconstruct images that simulate the appearance of scanning with a single energy, often called the effective energy. MonoE images are valuable because they can reduce beam-hardening artifacts and improve contrast resolution. By adjusting the energy level (typically ranging from 40 keV to 140 keV), clinicians can enhance the contrast between different tissues or reduce artifacts that might obscure diagnostic information. Clinicians can select different monoenergetic levels to optimize image contrast and clarity for specific clinical applications. For instance, lower energy levels can enhance contrast for soft tissues, while higher energy levels can be used to reduce metal artifacts. Reconstruction algorithms for generating MonoE images 444 of different energy levels are well known in the field. MonoE images 444 can be formed for example from a reconstruction algorithm applied to a weighted combination of the raw x-ray detector measurement data energy channels 404_n, or applied to a weighted combination of the material decomposed measurement data spectral channels 424_n (e.g. a weighted combination of Photo and Scatter channels). The weightings depend upon the monoenergetic level being targeted for the MonoE image 444.

Another type of material image 440 is a material map 446. A material map 446 represents the distribution of a particular material or substance within the scanned body. To generate a material map representing a particular material, a material decomposition procedure 410 may be selected which is configured to decompose the raw x-ray detector measurement data 402 into spectral channels which include at least one spectral channel which represents the effective contribution of the particular material to attenuation. Different material decomposition algorithms are available which decompose the raw multi-energy detector data into spectral channels corresponding to different sets or pairs of individual material components. Reconstruction can then be applied to the relevant material channel among the spectral channels 424_n of the material decomposed detector measurement data 422 to result in a reconstructed material map 446 representative of the spatial distribution of the particular material across a slice or volume of the scanned body.

Another type of material image 440 is the category which includes photoelectric effect images and Compton scatter images. These might be referred to as attenuation phenomenon images 448. A photoelectric effect image represents the effective contribution of the photoelectric effect (absorption) to x-ray attenuation as a function of position across a slice or volume of the scanned body. A Compton Scatter image represents the effective contribution of the Compton Scatter effect to x-ray attenuation as a function of position across a slice or volume of the scanned body. Known material decomposition algorithms exist which can be applied in the material decomposition procedure 410 which result in decomposition of the raw x-ray detector measurement data 402 into spectral channels 424_n which consist of a photoelectric effect channel 424_p (sometimes shortened to `photo channel') and a Compton scatter channel 424_s (sometimes referred to as 'scatter channel'). Reconstruction can then be applied to one or both of the scatter channel 424_s data and photo channel 424_p data to yield a photoelectric effect image and/or a Compton scatter image 448.

Optionally, there may further be performed a noise computation operation 450. Noise estimation in spectral CT involves quantifying the level of noise present in the reconstructed images. The output is a noise estimate 452 for each reconstructed material image 440. Noise estimation can be performed during or after the image reconstruction process. The purpose of this is to understand the extent of the noise so that it can be minimized, accounted for, or corrected. Noise computation methods in the context of spectral CT imaging is well known in the field.

The large variety of possible material images 440 that can be generated using spectral CT imaging results in a potentially large amount of data that needs to be stored or transmitted from the CT imaging system to an image viewer or image storage system (e.g. PACS system). This creates problems in terms of storage capacity requirements and data transfer capacity, including time taken for data transfer.

These problems can be mitigated partly by applying data compression techniques to the image data.

Data compression applied to an image can result in reduction of the matrix size of the image. Matrix size is the number of pixels in the reconstructed CT image grid. It is typically expressed as a two-dimensional array, represented by the number of rows and columns of pixels (e.g., 512 x 512, 1024 x 1024). For example, a matrix size of 512 x 512 means there are 512 pixels along the width (columns) and 512 pixels along the height (rows) of the image. A voxel (volumetric pixel) represents a volume element in the 3D reconstruction, which corresponds to the pixel area in 2D and the slice thickness. The voxel size is influenced by the matrix size, the field of view (FOV), and the slice thickness:
Matrix size directly influences the spatial resolution of the CT image. A larger matrix size (e.g., 1024 x 1024) provides higher spatial resolution. A smaller matrix size (e.g., 256 x 256) results in lower spatial resolution.

Commonly used CT scanners yield images with matrix sizes of 512 x 512 for routine diagnostic imaging. For specific applications requiring high spatial resolution (e.g., imaging of small bones, ear structures, or small vascular details), matrix sizes of 1024 x 1024 or higher may be used.

A variety of different image compression protocols and procedures are known in the field which can be used to reduce the matrix size of reconstructed CT images for storage or transmission. Two classifications are lossless and lossy compression.

Lossless compression reduces the file size without losing any information from the original image. When the image is decompressed, it is identical to the original, with no loss in quality or detail. Examples techniques include Run-Length encoding (RLE), Huffinan Coding, Lempel-Ziv-Welch (LZW). Lossless compression is commonly used in medical imaging (e.g. DICOM) because it avoids loss of image integrity.

Lossy compression reduces file size by removing some of the image data, which results in a loss of quality. The decompressed image is not identical to the original but is often visually similar. Lossy compression can achieve greater reduction in data size (matrix size) than lossless compression. Examples techniques include transform coding, quantization and wavelet transforms. Lossy compression is commonly used for web images, digital photography, and other uses where a slight loss in image integrity is acceptable to achieve reduced file size.

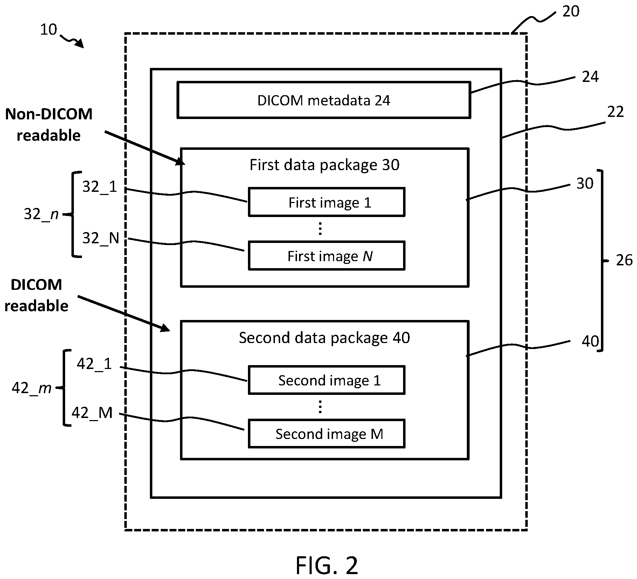

Fig. 2 illustrates schematically an example CT image dataset 10 in accordance with one or more embodiments of the invention. The CT image dataset 10 comprises post-reconstruction CT image data which includes one or more material images 440 formed from reconstruction 430 of spectral CT projection data 400 after material decomposition 410 has been applied.

The CT image dataset 10 is configured in a DICOM data structure 20.

The DICOM data structure 20 comprises a container 22. The container is, for example, a DICOM file. The DICOM data structure 20 further comprises DICOM metadata 24. The DICOM data structure further comprises a set of data packages 30, 40 containing image data. The set of data packages includes a first data package 30 and a second data package 40. Principles underlying DICOM data structures and methods for their compilation, starting from image data, have been described earlier in this disclosure.

The first data package 30 comprises (A) a set of one or more first images 32_1,... ,32_N. Each first image 32_n of the set of one or more first images is a post-reconstruction CT image 52 which is compressed in a first compression format 72. The first compression format 72 is a compression format which is not DICOM readable.

The second data package 40 comprises (B) a set of one or more second images 42_1,...,32_M. Each second image 42_m of the set of one or more second images is a post-reconstruction CT image 54 which is compressed in a second compression format 74. The second compression format is compression format which is DICOM readable.

A DICOM-readable compression format means that the second image is compatible with the DICOM standard.

A compression format which is not DICOM-readable means that each of the one or more first images is non-compatible with the DICOM standard.

In this disclosure, a first image as a category of image will be referred to generically with the reference numeral 32_n. In this disclosure a second image as a category of image will be referred to generically with the reference numeral 34_n.

Preferably there are a plurality of first images. Preferably there are fewer second images than first images. Preferably there is only a single second image and a plurality of first images.

In some embodiments, each of one or more second images 42_m may be an image formed from a reconstruction applied to a weighted combination of two or more spectral channels of the spectral CT projection data. With reference to Fig. 1, the two or more spectral channels may be two or more energy channels 404_n of the x-ray detector measurement data 402. Alternatively, the two or more spectral channels may be two or more spectral channels 424_n of the materially decomposed detector measurement data 422, i.e. of the CT projection data after material decomposition 410 has been applied. By way of example, and with reference to Fig. 1, at least one of the one or more second images 42_m may be a MonoE image 444, preferably a 70KV MonoE image) By way of further example, at least one of the one or more second images 42_m may be a pseudo-conventional image 442.

With reference to Fig. 1, in some embodiments, at least one of the one or more first images comprises a material map 446. The meaning of material map has been explained earlier in this disclosure with reference to Fig 1. A material map 446 is a representation of the spatial distribution of a particular material across a slice or volume of the scanned body.

Preferably the one or more first images includes a plurality of first images, each of which represents a different material map.

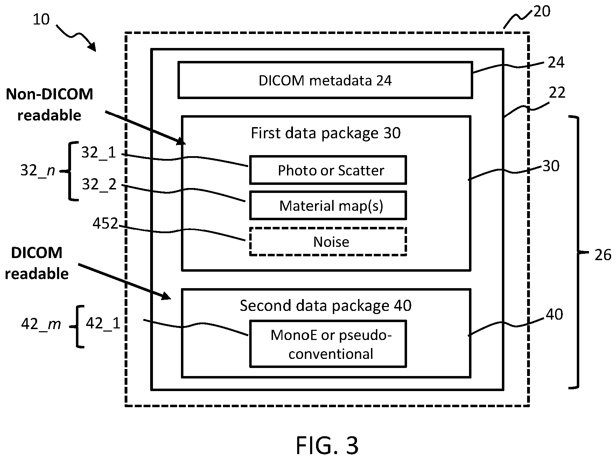

One preferred embodiment of the CT image data set is shown schematically in Fig. 3.

In this preferred embodiment, the one or more first images 32_n comprised by the first data package 30 include at least one of a photoelectric effect image and a Compton scatter image, i.e. at least one attenuation image 448, with reference to Fig. 1. Preferably, the set of one or more second images 42_m comprises only one of a photo image or a scatter image, to minimize data size. The one or more first images 32_n further include one or more material maps 446. The one or more second images 42_m include at least one of: a MonoE image 444 (preferably a 70KV MonoE image) and a pseudo-conventional image 442. Preferably the one or more second images 42_m includes only one of a MonoE image or a pseudo-conventional image, to minimize data size. Optionally, the first data package 30 may additionally contain a noise estimate 452 for the CT image data. Details regarding computation of a noise estimate have been outlined above with reference to Fig. 1.

The preferred embodiment of Fig. 3 has the following advantages. As will be explained later, a photo or scatter image in combination with a MonoE or pseudo-conventional image allows for reconstructing any desired material image 440. This is because both of these types of image are formed from a reconstruction applied to a weighted combination of two or more energy channels of the measurement data or two or more spectral channels of the CT projection data after material decomposition has been applied. This means that both of these types of images allow for decomposition into spectral components. In combination with one or other of a photo or scatter image, this then allows for reconstruction of any desired material image 440.

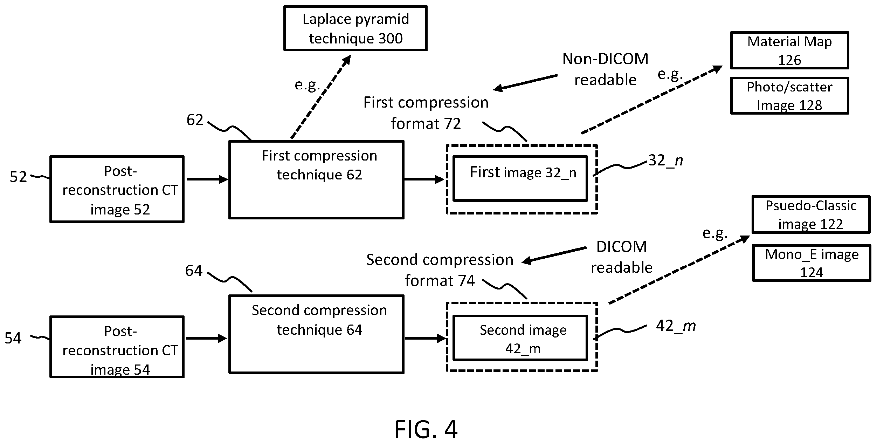

A corresponding method of forming a CT image dataset 10 is illustrated schematically in Fig. 4 to Fig. 6.

The method first comprises obtaining an initial version of one or more first images, wherein the initial versions of each of the one or more first images is a post-reconstruction CT image 52.

The method further comprises obtaining initial versions of one or more second images, wherein the initial version of each of the one or more second images is a post-reconstruction CT image 54.

With reference to Fig. 4, the method further comprises applying a first compression technique 62 to the initial version 52 of each of the one or more first images to thereby compress each of the one or more first images 32_n in a first compression format 72. The first compression format 72 is a compression format which is not DICOM-readable.

The method further comprises applying a second compression technique 64 to the initial version 54 of each of the one or more second images to thereby compress each of the one or more second images 42_m in a second compression format 74. The second compression format 74 is a compression format which is DICOM-readable.

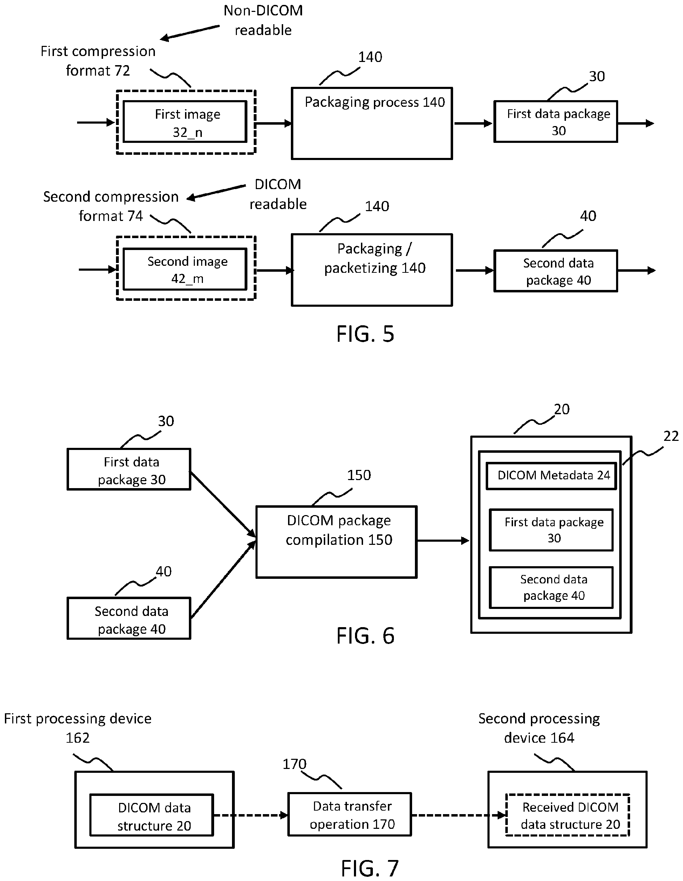

With reference to Fig. 5, a packaging process 140 is applied to the one or more first images 32_n in the first compression format 72. The packaging process comprises packaging the one or more first images 32_n to form a first data package 30. The packaging process 140 is further applied to the one or more second images 42_m in the second compression format 74. The packaging process comprises packaging the one or more second images 42_m to form a second data package 40.

With reference to Fig. 6, the method further comprises applying a DICOM compilation procedure 150. The DICOM compilation procedure 150 comprises compiling a DICOM data structure 20. The DICOM data structure corresponds to the DICOM data structure of Fig. 2. The DICOM data structure comprises a container 22, DICOM metadata 24, the first data package 30 and the second data package 40.

The procedure for compiling a DICOM data structure has been described earlier in this disclosure.

With regards to the first compression format 72 and second compression format 74, in some embodiments, the first and second compression formats may be such that each of the one or more second images 42_m is compressed to a lesser or lower level of compression than each of the one or more first images 32_n . A lesser or lower level of compression means that the second compression format results in less reduction in the data size of the image compared to the first compression format. A lesser level of compression may mean that the second compression format corresponds to a smaller matrix size for each of the one or more second images 42_m compared to the matrix size for the one or more first images 32_n.

In some embodiments, the first compression technique 62 may be a lossy compression technique so that the first compression format 72 is a lossy compression format. The second compression technique 64 may be non-lossy compression technique so that the second compression format 74 is a non-lossy compression format.

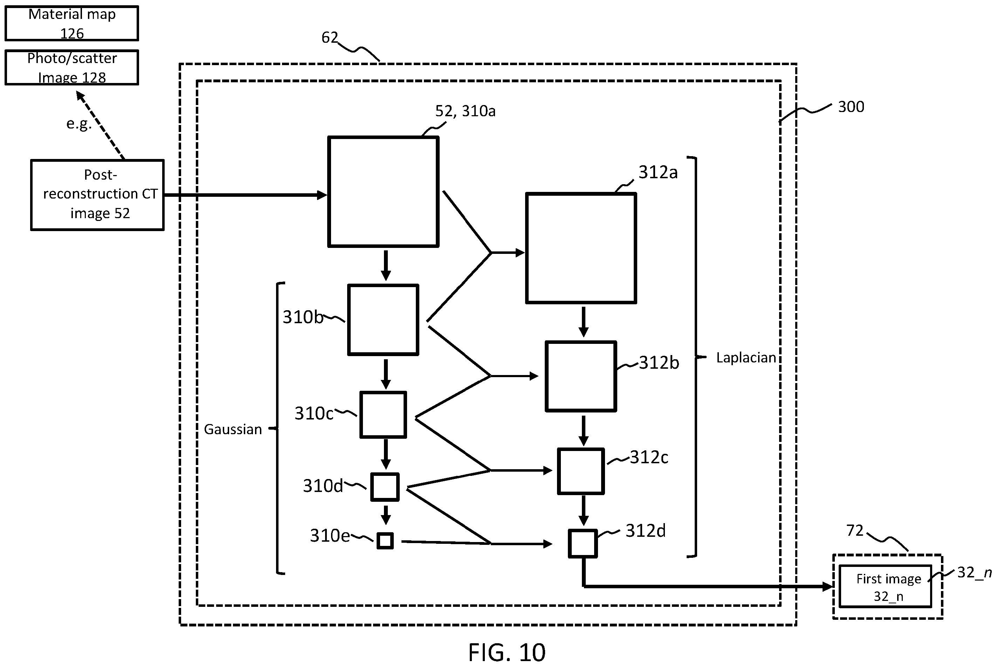

One specific example compression format and associated compression method will be discussed in detail later with reference to Fig. 10.

With regards to the one or more post-reconstruction CT images 52 which form the one or more first images after compression, options with regards to these have already been discussed. With regards to the one or more post-reconstruction CT images 54 which form the one or more second images after compression, options with regards to these have also already been discussed.

One of the purposes of compressing and packaging the CT image data in a DICOM data structure is to facilitate efficient storage and/or transmission across a data communication channel to one or more further devices, e.g. a storage server and/or an image viewing console.

Accordingly, with reference to Fig. 7, in some embodiments, the steps for compiling the CT image dataset 10 configured in the DICOM data structure 20 which are represented in Fig. 4-Fig. 6 may be performed at a first processing device 162, and wherein the method further comprises a data transfer operation 170 which comprises transferring the CT image dataset 10 configured in the DICOM data structure 20 to a second processing device 164.

By way of example, the first processing device may be a data processing device which is local to a CT image system, the CT imaging system including a CT scanner. The first processing device may be configured to process the raw spectral CT projection data 400 and to generate one or more material images 440. The first processing device may be configured to perform some or all of the sequence of processing steps outlined in Fig. 1 and described earlier in the disclosure.

By way of example, the second processing device may be a processing device which is local to, or comprised by, a data storage system and/or one or more image display systems (e.g. an image viewer device). A data storage system may be a PACS data storage system. An image display system may be a DICOM image viewer device, and/or may be a proprietary image viewer device.

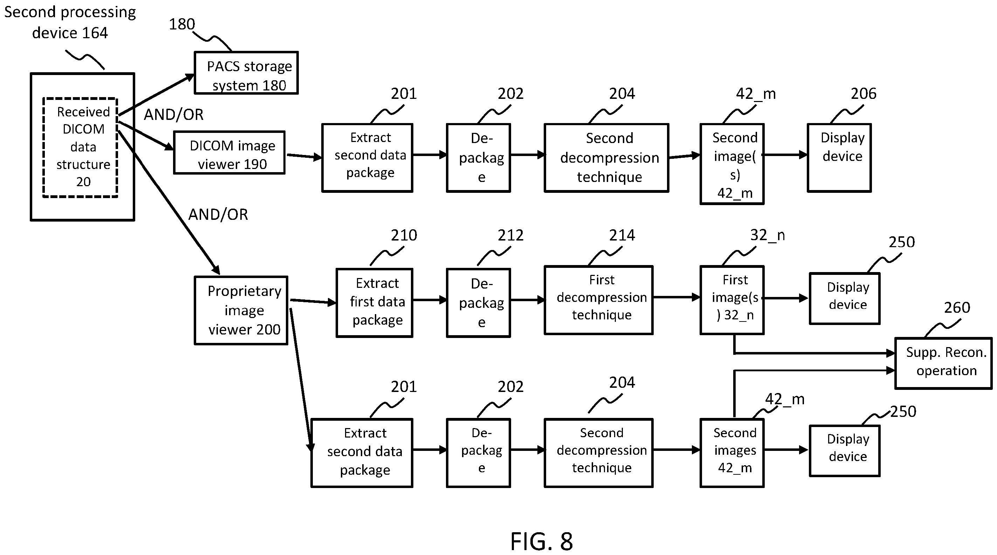

Reference is made to Fig. 8, which illustrates schematically various options for how the received DICOM data structure 20 may be used at the second processing device 164.

In some embodiments, the method may comprise, at the second processing device 164, receiving the DICOM data structure 20 and storing the received DICOM data structure in a DICOM-compatible PACS storage system 180. The principles of PACS storage systems have been discussed earlier in this disclosure. The configuring of the CT image dataset 10 in a DICOM data structure 20 has the result that the CT image dataset 10 is compatible standard PACS storage systems since these utilize the DICOM standard. For example, the PCAS system can read the DICOM metadata 24 in the DICOM data structure 20 and use this to store and appropriately tag the contents of the DICOM data structure in the PACS system.

Additionally or alternatively, the method may comprise, at the second processing device 164, receiving the DICOM data structure 20 and displaying at least one of the one or more second images 42_m using a DICOM image viewer 190. A DICOM image viewer means a device configured to read DICOM-compatible image data which is stored in a received DICOM data structure and which is operable to display one or more images encoded in the read DICOM-compatible image data using a display device comprised by the DICOM image viewer. DICOM-compatible image data means image data encoding one or more images which are in a DICOM-compatible compression format.

The displaying using the DICOM image viewer 190 may comprise extracting the at least one of the one or more second images 42_m. The extracting may comprise extracting 201 the second data package 40 from the DICOM data structure 20, and de-packaging 202 the one or more second images 42_m from the second data package 40. The displaying may further comprise performing a second decompression technique 204 which comprises decompressing at least one of the de-packaged one or more second images 42_m so that it is no longer in the second compression format 74. The second decompression technique 204 may be an inverse of the second compression technique 64. The decompressed at least one second image 42_m may then be displayed on a display device 206 of the DICOM image viewer.

Additionally or alternatively, the method may comprise, at the second processing device 164, receiving the DICOM data structure 20 and displaying at least one of the one or more first images 32_n using an image viewer 200 which is operable to read and display images which are in a compression format which is not DICOM readable. This image viewer 200 may be referred to as a proprietary image viewer 200. The proprietary image viewer 200 may be operable to read and display both: images which are in compression format which is DICOM readable and images which are in a compression format which is not DICOM readable. The displaying of at least one of the one or more first images at the proprietary image viewer 200 may comprise extracting 210 the first data package 30 from the DICOM data structure 20, and de-packaging 212 the one or more first images 32_n from the second data package 40. The displaying may further comprise performing a first decompression technique 214 which comprises decompressing at least one of the de-packaged one or more first images 32_n so that it is no longer in the first compression format 72. The first decompression technique 214 may be an inverse of the first compression technique 64. The decompressed at least one first image 32_n may then be displayed on a display device 250 of the proprietary image viewer 200.

By configuring the CT image data in a DICOM data package that includes both DICOM-readable images (i.e. images in a compression format which is DICOM readable) and non-DICOM readable images (i.e. images in a compression format which is not DICOM readable), this improves operability, since the CT image data, when so packaged, can be handled and read by both conventional DICOM image viewers (which are standard in most hospitals), but also enhanced proprietary image viewers. The enhanced proprietary image viewers are able to access additional image data, compressed in the first compression format. The first compression format can be for example a higher degree of compression (i.e. greater reduction in data size) than the second compression format, so that its additional inclusion does not increase the overall size of the CT image data set too greatly. However, its inclusion allows additional information and insights to be obtained where there is available an image viewer which is operable to read image data compressed in a non-DICOM compatible compression format.

As already recited, where the method involves use of a proprietary image viewer 200, the optionally the proprietary image viewer 200 may be operable to read and display images both in a DICOM-readable compression format and images in a non DICOM-readable compression format. This is further schematically represented in Fig. 7 which shows that the method may further comprise extracting and displaying one or more of the second images 42_m on a display device 250 of the proprietary image viewer 200. This may involve a similar series of steps as were outlined previously in respect of the DICOM image viewer 190. The displaying of at least one of the one or more second images may comprise extracting the at least one of the one or more second images 42_m. The extracting may comprise extracting 201 the second data package 40 from the DICOM data structure 20, and de-packaging 202 the one or more second images 42_m from the second data package 40. The displaying may further comprise performing a second decompression technique 204 which comprises decompressing at least one of the de-packaged one or more second images 42_m so that it is no longer in the second compression format 74. The second decompression technique 204 may be an inverse of the second compression technique 64. The decompressed at least one second image 42_m may then be displayed on a display device 250 of the proprietary image viewer.

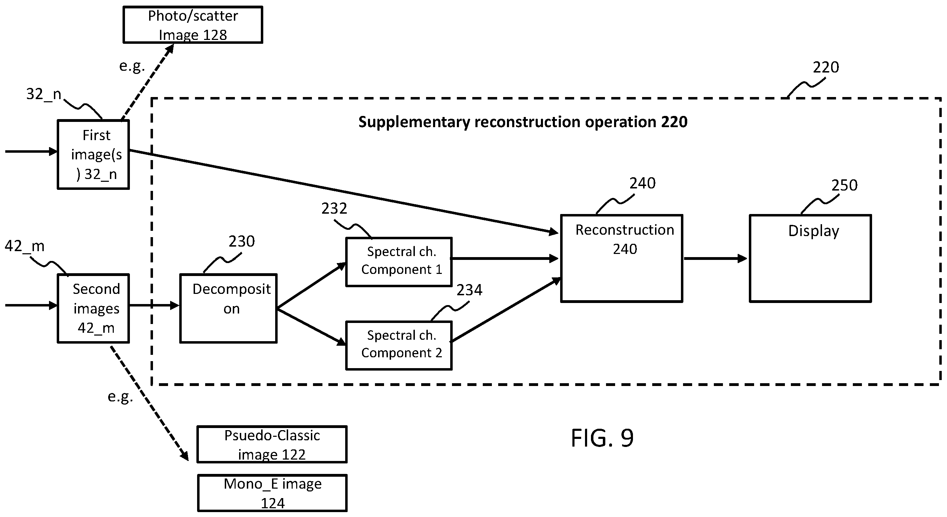

With reference to Fig. 8 and Fig. 9, optionally, in some embodiments, the method may comprise a supplementary reconstruction operation 260 utilizing the extracted and decompressed one or more first images 32_n and the extracted and decompressed one or more second images 42_m. The supplementary reconstruction operation is for reconstructing one or more supplementary images, different to any of the one or more first images and one or more second images, based on processing of at least one of the one or more first images and at least one of the one or more second images. The processing may comprise a spectral decomposition operation 230 which comprises decomposing at least one of the one or more second images 42_m into two or more projection domain spectral channel components 232, 234. For example, the two or more projection domain spectral channel components may comprise a first spectral channel component 232 and a second spectral channel component 234. With reference to Fig. 1 described earlier, in some embodiments, the two or more projection domain spectral channels may be two or more energy channels 404_n of the x-ray detector measurement data 402. In some embodiments, the two or more projection domain spectral channels may be two or more of the spectral channels 424_n of materially decomposed detector measurement data 422, i.e. two or more of the spectral channels 424_n of the CT projection data after material decomposition 410 has been applied. By way of one example, the first spectral channel component 232 may be a Compton Scatter spectral channel components, and the second spectral channel component 234 may be a Photoelectric effect spectral channel component.

By way of example, if at least one of the one or more second images 42_m is a MonoE image, this is formed by applying reconstruction to a weighted combination of Photo and Scatter spectral channels of the materially decomposed detector measurement data 422. Thus, if the weightings used in forming the MonoE image are known, it is possible to decompose the MonoE image back into the individual projection domain Photo and Scatter spectral channels originally used in forming the image. The weightings may be stored in the DICOM data package. The weightings may be known information, for example stored locally at the proprietary image viewer 200. The weightings may be derivable from a known Monoenergetic energy level represented by the relevant MonoE image. The same principle can be applied to decompose the image into a different one or more projection domain spectral channels than Photo and Scatter channels.
Furthermore, the same principle can be applied for a different type of image than a MonoE image. Thus, in similar fashion, a pseudo-conventional image can be decomposed into two or more projection domain spectral channels.

The supplementary reconstruction operation 220 may further comprise processing the decomposed projection domain spectral channels 232, 234 of the at least one second image in combination with the first image 32_n with a reconstruction operation 240. The reconstruction operation is able to reconstruct any desired class of image which can be reconstructed using the two or more spectral channels of the second image in combination with information derivable from the first image.

With regards to the first compression technique 62 discussed with reference to Fig. 4 earlier in this disclosure, in some embodiments, the first compression technique comprises a multi-scale image decomposition procedure. For example, the first compression technique comprises a Laplacian pyramid decomposition procedure. By way of example, the first compression format 72 (referenced in Fig. 3) may comprise one or more decomposition levels of a Laplacian pyramid decomposition applied to a starting CT image 52. By way of one example, the first compression format 72 may comprise a lowest resolution decomposition level of the Laplacian pyramid decomposition applied to the starting CT image 52. Multi-scale decomposition techniques such as Laplacian pyramid decomposition result in lossy compression of the starting image, for reasons that will now be explained. Images compressed using this type of technique are not readable by DICOM-standard systems. However, this type of lossy compression permits significantly greater reduction in the image data size compared with compression formats which are DICOM-compatible. It is proposed in accordance with some embodiments to compress at least one of the one or more first images using a lossy compression technique such as Laplacian pyramid decomposition, but to compress the one or more second images using a compression format that does not reduce the data size to as greater an extent but which is readable by DICOM standard systems.

To explain further, and by way of background, multi-scale decomposition in image processing is a technique used to analyze images at different scales or resolutions. The basic concept of multi-scale decomposition is to create a series of images, each representing an original image at a different scale or level of detail. This may in general be achieved by repeatedly smoothing and subsampling the image, which results in a set of images ranging from coarse (low resolution) to fine (high resolution) scales. Some example types of multi-scale decomposition include pyramid decomposition, wavelet decomposition, Gaussian pyramid decomposition, and Laplacian Pyramid decomposition.

In general, in pyramid decomposition: the image is recursively reduced in size, usually by a factor of 2 each time, to create a stack of progressively smaller images, notionally forming a pyramid shape. Each level of the pyramid represents the image at a different scale. Each level of the pyramid might be referred to a decomposition level. Each level of the pyramid might be referred to as a `spatial sub-band' in the sense that it represents a particular spatial frequency (i.e. resolution) level.

Gaussian pyramid decomposition is type of pyramid decomposition in which each level is formed by smoothing the image with a Gaussian filter and then subsampling. This process is repeated to generate a series of reduced images. Each reduced image might be referred to a decomposition level of the Gaussian pyramid. Each level of the pyramid might be referred to as a `spatial sub-band' in the sense that it represents a particular spatial frequency (i.e. resolution) level.

Laplacian pyramid decomposition is a more sophisticated form of pyramid decomposition. In the Laplacian pyramid, each level is formed by subtracting levels (otherwise known as 'sub-bands') of a Gaussian pyramid decomposition from one another. This therefore emphasizes the differences (edges) between scales. The process involves creating a Gaussian pyramid first and then using it to construct the Laplacian pyramid.

For performing a Laplacian pyramid decomposition, a first step is to create a Gaussian pyramid by repeatedly applying smoothing (e.g. Gaussian blur) and downsampling the image. Once the Gaussian pyramid is constructed, for each given level in the Gaussian pyramid, a subsequent level is interpolated back to a same scale size as the given level and subtracted from the given level. In other words, for each level in the Gaussian pyramid, there is derived a full-resolution `difference image' representing a difference between the given level and an up-sampled version of that level. Each resulting difference image forms a level of the Laplacian pyramid. Each level of the Laplacian pyramid represents the image details lost at the next level of the Gaussian pyramid. The lowest level of the Laplacian pyramid is thus a small-matrix smooth difference image.

Each level of the Laplacian pyramid highlights features lost due to smoothing at that scale. For instance, the first level shows the finest details (such as edges), and the subsequent levels represent progressively coarser details.

It is possible to reconstruct the original image from the Laplacian pyramid by starting from the smallest image, up-sampling it, and adding it back to the next level of the pyramid. This process is repeated until the largest level is reached, this corresponding to the original image.

The process of performing the Laplacian pyramid decomposition in the context of one or more embodiments of the present invention is illustrated schematically in Fig. 10. Fig. 10 schematically illustrates an example Laplacian pyramid based compression procedure 300. The first compression technique 62 may comprise the Laplacian pyramid based compression procedure 300 in accordance with one or more embodiments

Fig. 10 shows (left) first performing a Gaussian pyramid deconstruction by recursively downsampling and blurring a starting CT image 52 to result in a pyramid decomposition formed of a plurality of Gaussian decomposition levels 310a-3 10e. The top level 310a of the decomposition is identical to the original image-to-be-enhanced 52. Subsequently, each neighboring pair of decomposition levels of the Gaussian decomposition are used to form one of the levels of the Laplacian pyramid decomposition. The smaller scale level of each neighboring pair of levels is up-sampled to match the scale of the higher scale level of the pair and then the two are subtracted to yield one level of the Laplacian pyramid. The resulting set of decomposition levels 312a-312d of the Laplacian decomposition are schematically illustrated.

The number of decomposition levels of the Gaussian and Laplacian pyramids may be determined in advance, and may be a pre-determined figure or may vary depending upon circumstances. The number of decomposition levels is determined by the so-called cut-off resolution. The cut-off resolution is indicative of the lowest-resolved smooth (Gaussian) volume which is worthy to be retained for the down-sampled images. It may be selected in advance. It may be a fixed threshold derived from knowledge of the spectral processing chain. Alternatively, it may be determined from scan-specific estimates derived from the image content (e.g. physical line pair measurements on a phantom, or typical anatomical structures for a certain exam type).

With reference to Fig. 10, in some embodiments, the first compression format 72 may comprise a lowest resolution decomposition level 312d of the Laplacian pyramid decomposition applied to the starting CT image 52.

To decompress the image, the first decompression technique 214 may comprise the standard Laplacian reconstruction approach outlined above.

For example, an initial version 52 of each first image 32_n may have a full matrix size of 1024 with 0.25 mm sampling. Each first image is decomposed into a recursive series of spatial sub-bands, each corresponding to a reduced resolution level. The matrix size is halved at each successive sub-band. This thereby results in a Gaussian pyramid decomposition of the initial version of the relevant first image. The Laplacian (difference image) of the lowest level of the Gaussian pyramid is retained. In one preferred set of embodiments, the set of one or more first images includes at least one of a photo image and a scatter image (i.e. an attenuation image 448, with reference to Fig. 1), and may include one or more material maps 446. The lowest resolution level 312d of the Laplacian pyramid decomposition may be retained for the photo or scatter image and/or the material maps of interest.

In some embodiments, prior to applying the Laplacian pyramid decomposition, the first compression technique 62 may comprise converting the initial version 52 of the first image 32_n which is to be compressed from the logarithmic space of the Hounsfield densities to (positive) mass values (HU+1000). After performing the standard Laplacian pyramid reconstruction, the first decompression technique 204 may comprise re-converting the mass values to Hounsfield density HU-values. This has the effect of increasing contrast of edges. An edge which has weak contrast in the conventional image volume is effectively scaled up to stronger contrast for e.g. a low-keV mono-energy image, because an adding of a Laplacian edge in log-space translates to a multiplication in HU-space.

In a preliminary C#-implementation tested by the Applicant, decomposition time for a 3D image volume was observed as 4 seconds for a 1024² × 640 volume into Laplacian pyramidal representation. A re-composition time of two seconds was observed for returning to full matrix.

In some embodiments, the first compression technique 62 may further comprise, subsequent to applying the Laplacian pyramid decomposition, applying a further compression algorithm, this having the effect of further reducing the size of the relevant image being compressed. For example, the lowest resolution level 312d of the Laplacian pyramid decomposition may be further processed with a lossless compression technique such as run-length encoding or entropy encoding.

In some embodiments (but not illustrated in Fig. 9), the method may further comprise applying a Laplacian pyramid decomposition to at least one of the one or more second images. In some embodiments (but not illustrated in Fig. 9), the DICOM data structure 20 may be compiled so as to further include a data representation of all of the decomposition levels of a Laplacian pyramid decomposition of at least one of the one or more second images 42_m. For example, the one or more second images 42_m may comprise a pseudo-conventional image 442 and/or a MonoE image 444, and the complete set of Laplacian pyramid decomposition levels (`difference images') may be computed and included in the DICOM data structure 20 for the pseudo-conventional image and the MonoE image.

During decompression of the compressed one or more first images 32_n, i.e. reconstruction of the first image to the original matrix size, the decomposition levels of the second image can be used to reconstruct a better resolution version of the original first image, i.e. it can be used to reduce some of the data loss incurred in the original downsampling process. For a compressed first image, the corresponding lowest resolution level of the Laplacian pyramid may be recursively up-sampled using the pyramidal Laplacian band volumes of the second image (e.g. MonoE or pseudo-conventional image). The second image is assumed to have the best spatial fidelity. The upsampling is performed until the full matrix size is re-established. (E.g. for a 1024 matrix of 0.25 mm grid sampling, the *physical* spatial resolution of the spectral information may be only 1 mm, which is corresponding to the sub-band volume of the 256 matrix. This allows a compression factor of 64 = 4×4×4, meaning that only 1.6% additional space is needed for each spectral map in addition to the conventional image volume.)

As an alternative to the Laplacian pyramid decomposition, the first compression technique 62 may instead comprise application of a Fast Wavelet Transform (FWT). Indeed, the Laplacian pyramid is known to be less space-efficient (sparse) than the Fast Wavelet Transform (FWT). The pyramid decomposition requires 4/3 of the original data size, i.e., 33% more space than the original image, while the FWT pyramid can decompose entirely inside the original space. However, the Laplacian pyramid is known to give a better visual perception after modifications (enhancements) have been applied to the sub-bands before re-composition, avoiding typical wavelet-artefacts. The 1/3 additional volume (compared to the full original volume) is required only in computer memory, not in persistent storage, as the Laplacian pyramid can be efficiently re-computed on the fly.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A CT image dataset (10) configured in a DICOM data structure (20), the CT image dataset comprising post-reconstruction CT image data generated from spectral CT projection data (400), the spectral CT projection data comprising x-ray measurement data (402) for a plurality of energy channels (404_n), and the post-reconstruction CT image data including one or more material images (440) formed from reconstruction (430) of the spectral CT projection data after material decomposition (410) has been applied,
wherein the DICOM data structure (20) comprises:
a container (22);
DICOM metadata (24); and
a set of data packages (30, 40), the set of data packages including a first data package (30) and a second data package (40);
wherein the first data package (30) comprises (A) a set of one or more first images (32_n), wherein each first image (32_n) of the set of one or more first images is a post-reconstruction CT image (52) which is compressed in a first compression format (72), wherein the first compression format (72) is not DICOM readable; and
wherein the second data package (40) comprises (B) a second image (42_m), wherein the second image is a post-reconstruction CT image (54) which is compressed in a second compression format (74), wherein the second compression format is DICOM readable.

2. The CT image dataset of claim 1, wherein the first and second compression formats are such that the second image is compressed to a lesser level of compression than each of the one or more first images.

3. The CT image dataset of claim 1, wherein the second image is an image formed from a reconstruction applied to a weighted combination of two or more spectral channels of the spectral CT projection data, wherein the two or more spectral channels are two or more energy channels of the measurement data or two or more spectral channels of the CT projection data after material decomposition has been applied.

4. The CT image dataset of any preceding claim, wherein the first compression format is a lossy compression format.

5. The CT image dataset of any preceding claim,
wherein the second image is a MonoE image; or
wherein the second image is a pseudo-conventional CT image formed from a reconstruction applied to a weighted combination of two or more of the measurement data energy channels.

6. The CT image dataset of any preceding claim, wherein at least one of the set of one or more first images is one of: a Compton Scatter image or a Photoelectric effect image.

7. The CT image dataset of any preceding claim, wherein at least one of the one or more first images comprises a material image / material map, and preferably wherein the one or more first images include a plurality of different material images / material maps.

8. The CT image dataset of any preceding claim, wherein the first compression format comprises one or more decomposition levels of a Laplacian pyramid decomposition applied to a starting CT image.

9. The CT image dataset of claim 8, wherein the first compression format comprises a lowest resolution decomposition level of the Laplacian pyramid decomposition applied to the starting CT image.

10. The CT image dataset of claim 9, wherein the data structure further includes all decomposition levels of a Laplacian pyramid decomposition of the second image.

11. A method of forming a CT image dataset, the CT image dataset comprising post-reconstruction CT image data generated from spectral CT projection data, the spectral CT projection data comprising x-ray measurement data for a plurality of energy channels, and the post-reconstruction CT image data including one or more material images formed from reconstruction of the CT projection data after material decomposition has been applied,
the method comprising:
a) obtaining one or more first images, wherein each of the one or more first images is a post-reconstruction CT image
b) obtaining a second image; wherein the second image is a post-reconstruction CT image;
c) compressing the one or more first images in a first compression format, wherein the first compression format is not DICOM-readable;
d) compressing the second image in a second compression format, wherein the second compression format is DICOM-readable;
e) packaging the one or more first images to form a first data package;
f) packaging the second image to form a second data package; and
g) compiling a DICOM data structure, wherein the DICOM data structure includes a container, DICOM metadata, the first data package and the second data package.

12. The method of claim 11,
wherein steps (a)-(g) are performed at a first processing device; and
wherein the method further comprises transferring the DICOM data structure to a second processing device.

13. The method of claim 12, wherein the method further comprises, at the second processing device:
receiving the DICOM data structure; and
extracting the one or more first images, decompressing at least one of the one or more first images, and displaying at least one of the one or more first images using an image viewer which is operable to read and display images which are in a compression format which is not DICOM readable; AND/OR
extracting the second image, decompressing the second image, and displaying the second image using a DICOM image viewer;
AND/OR
storing the received DICOM data package in a DICOM-compatible PACS system.

14. The method of any of claims 11-13, further comprising
extracting the second image from the data structure; and
decomposing the second image into two or more projection domain spectral channel components, wherein the two or more projection domain spectral channels are two or more energy channels of the measurement data or two or more spectral channels of the CT projection data after material decomposition has been applied.

15. The method of claim 14,
wherein the second image is an image formed from a reconstruction applied to a weighted combination of two or more spectral channels of the spectral CT projection data, wherein the two or more spectral channels are two or more energy channels of the measurement data or two or more spectral channels of the CT projection data after material decomposition has been applied;
wherein the set of one or more first images (32_n) includes a photoelectric effect image or a Compton Scatter image
wherein the method further comprises a supplementary reconstruction operation comprising processing the second image and the photoelectric effect or scatter image to yield a material map image.
